# EUROPÄISCHE PATENTSCHRIFT

(11) **EP 1 370 641 B1**
(45) Veröffentlichungstag und Bekanntmachung des Hinweises auf die Patenterteilung: **16.08.2006**
(21) Anmeldenummer: 01986605.2
(22) Anmeldetag: 11.10.2001
(51) Int. Cl.: C12N 5/06, A61P 37/06, A61P 35/02

(54) **VERWENDUNG VON STAMMZELLEN UND CD6-DEPLETIERTEN STAMMZELLEN ZUR TOLERANZINDUKTION GEGENÜBER ALLOGENEN TRANSPLANTATEN UND/ODER ZUR LEUKÄMIEBEHANDLUNG**
USE OF STEM CELLS AND CD6-DEPLETED STEM CELLS FOR INDUCING TOLERANCE OF ALLOGENIC TRANSPLANTS AND/OR FOR TREATING LEUKAEMIA
UTILISATION DE CELLULES SOUCHES ET DE CELLULES SOUCHES DEPOURVUES DE CD6 POUR INDUIRE LA TOLERANCE VIS-A-VIS DE GREFFONS HOMOLOGUES ET / OU POUR LE TRAITEMENT DE LA LEUCEMIE

(30) Priorität: 11.10.2000 DE 10050334
(43) Veröffentlichungstag der Anmeldung: 17.12.2003
(73) Patentinhaber: GSF-Forschungszentrum für Umwelt und Gesundheit GmbH, 85764 Neuherberg (DE)
(72) Erfinder: KOLB, Hans-Jochen, 80809 München (DE); GÜNTHER, Wolfgang, 82152 Planegg (DE)
(74) Vertreter: Behnisch, Werner
(86) Internationale Anmeldenummer: PCT/EP2001/011776
(87) Internationale Veröffentlichungsnummer: WO 2002/030433

(56) Entgegenhaltungen:
- R. E. CHAMPLIN ET AL: "T-cell depletion of bone marrow transplants for leukemia from donors other than HLA-identical siblings: advantage of T-cell antibodies with narrow specificities" BLOOD, Bd. 95, Nr. 12, 15. Juni 2000 (2000-06-15), Seiten 3996-4003, XP002230453 in der Anmeldung erwähnt
- R. J. SOIFFER ET AL: "CD6-Depleted Allogeneic Bone Marrow Transplantation for Acute Leukemia in First Complete REmission" BLOOD, Bd. 89, Nr. 8, 15. April 1997 (1997-04-15), Seiten 3039-3047, XP002230454 in der Anmeldung erwähnt
- A. J. BARRETT ET AL: "Effect of nucleated marrow cell dose on relapse and survival in identical twin bone marrow transplants for leukemia" BLOOD, Bd. 95, Nr. 11, 1. Juni 2000 (2000-06-01), Seiten 3323-3327, XP002230455 in der Anmeldung erwähnt
- R. E. CHAMPLIN ET AL: "Blood stem cells compared with bone marrow as a source of hematopoietic cells for allogeneic transplantation" BLOOD, Bd. 95, Nr. 12, 15. Juni 2000 (2000-06-15), Seiten 3702-3709, XP002230456

## Beschreibung

Die vorliegende Erfindung betrifft die Verwendung einer Zusammensetzung, enthaltend Stammzellen sowie CD6-depletierte Stammzellen zur Toleranzinduktion gegenüber allogenen Transplantaten und/oder zur Behandlung von Blut-, Immun- und Krebserkrankungen zur zeitlich abgestuften Applikation, wobei zunächst die Stammzellen und anschließend die CD6-depletierten Stammzellen appliziert werden. Die vorliegende Erfindung betrifft außerdem ein Kombinationspräparat, das Stammzellen und CD6-depletierte Stammzellen enthält.

### Hintergrund der Erfindung

Die Transplantation von hämatopoetischen Stammzellen und Knochenmark ist Stand der Technik. Dabei werden zunächst durch eine Vorbehandlung wie z.B. mittels Chemotherapie oder Bestrahlung immunkompetente Zellen des Empfängers in ausreichendem Maße zerstört, um das Anwachsen der Spenderzellen einschließlich Stammzellen und immunkompetenten Zellen zu ermöglichen. Allgemein wird die Stammzelltransplantation bei Erkrankungen mit Funktionsausfall des Knochenmarks eingesetzt, beispielsweise im Rahmen der Therapie von akuter Leukämie, aber auch bei anderen Blut- und Immunerkrankungen sowie bei Krebserkrankungen. Dieses Spektrum der zu therapierenden Erkrankungen beruht auf der Tatsache, dass es sich in allen Fällen um Erkrankungen handelt, die auf einer Fehlfunktion der Blutbildung beruhen. So können Stammzellen bei nicht malignen Erkrankungen des Blutes (wie z.B. Schwere Aplastische Anämie (SAA), Aplastische Anämie, Sichelzellanämie, Thalasämie), Erkrankungen des Immunsystems (Multiple Sklerose(MS), Rheuma(CP) Sklerodermie) sowie bei malignen Blutkrankheiten (akute und chronische Leukämien myeloischen und lymphatischen Ursprunges) eingesetzt werden. Üblicherweise werden dabei dem Spender Stammzell-haltige Präparate wie Knochenmark und Blutleukozyten entnommen und dem Empfänger intravenös verabreicht. Eine solche Transplantation von Stammzellen kann Toleranz gegenüber Organen wie Herz, Lunge etc. sowie gegenüber Blutstammzellen des Stammzellspenders herbeiführen (1). Ein Nachteil einer solchen Stammzelltransplantation ist jedoch, daß einerseits das Transplantat des Patienten abgestoßen werden kann, andererseits immunkompetente Zellen des Transplantates den Empfänger im Wege einer Graft-versus-Host Krankheit (GVH), bei der T-Zellen des Transplantats Zellen des Empfängers als fremd erkennen, angreifen und schädigen können. Im Gegensatz zur Transplantation solider Organe kann bei der Stammzelltransplantation nach Monaten oder Jahren die immunsuppressive Behandlung abgesetzt werden, ohne daß eine Abstoßung oder eine GVH-Reaktion auftritt. Man geht von einer gegenseitigen Immuntoleranz aus.

Zur Verhinderung bzw. Abschwächung der Graft-versus-Host Reaktion werden häufig immunkompetente Zellen, insbesondere T-Lymphozyten, aus dem Transplantat entfernt. Dabei kommt es allerdings nicht selten zur Abstoßung des Transplantates (2), im Fall der Leukämiebehandlung treten vermehrt Rückfälle der Krankheit auf. Die Rückfälle bei der Leukämiebehandlung sowie die Abstoßung des Transplantates sind vermutlich darauf zurückzuführen, daß im Transplantat Spender T-Zellen fehlen, die in der Lage sind, die nach der Vorbehandlung des Empfängers übrig gebliebenen T-Zellen zu eliminieren (3). Das Risiko derartiger Komplikationen ist selbst bei HLA-Identität (Gewebemerkmalidentität) von Spender und Empfänger nicht gering, bei HLA-Differenzen jedoch sehr hoch.

Die Stammzelltransplantation zur Toleranzinduktion gegenüber Transplantaten bzw. zur Toleranzerhaltung wurde ursprünglich mit der Überlegung durchgeführt, daß das Immunsystem des Patienten durch das des Stammzellspenders ersetzt wird, welches das transplantierte Organ nicht angreift. Sobald ein Organismus daher fremde Stammzellen angenommen hat, wird dieser Organismus auch andere Organe annehmen (4). Da die Stammzelltransplantation über HLA-Differenzen hinweg allerdings sehr risikoreich ist, bleibt diese Methode derzeit nicht zur Toleranzinduktion gegen transplantierte Organe einsetzbar.

Beim Hund konnte gezeigt werden, daß durch T-Zelldepletion des Knochenmarks in besonderen Spender-Empfänger Kombinationen die Vermeidung einer starken Graft-versus-Host Reaktion möglich ist. In Kombinationen von DLA-homozygoten Spendern und DLA-heterozygoten Empfängern ist die Reaktion in Host-versus-Graft Richtung im Gegensatz zur GVH-Richtung schwach, es entsteht ein kompletter Chimärismus mit anhaltender Toleranz über einen unterschiedlichen DLA-Haplotyp hinweg (5). Dagegen führen starke DLA-Differenzen des heterozygoten Spenders, fast immer zur Abstoßung und erlauben keine beidseitige Toleranz.

Es existieren bereits verschiedene Versuche und Möglichkeiten, eine Abstoßung aufgrund von HLA-Differenzen beim Menschen zu verhindern bzw. zu verringern:

Beispielsweise ist bekannt, daß die Verwendung großer Mengen CD34-positiver Blutstammzellen, bei Erwachsenen (6) und Kindern (7) eine Toleranz induzieren kann. Bei großen Spendern und kleinen Empfängern, wie das bei der Transplantation von Eltern auf Kinder häufig der Fall ist, kann man Blutstammzellen in ausreichenden Mengen gewinnen. Bei ungünstigeren Größenverhältnissen wird die Gewinnung von Blutstammzellen in ausreichendem Maße problematisch.

Als weitere Methode zur Toleranzinduktion über HLA-Differenzen hinweg wurde die Knochenmarktransplantation in Verbindung mit einer Kombination von Cyclophosphamid, Bestrahlung des Thymus und Antithymozytenglobulin (ATG) vorgeschlagen (8). Bei Patienten mit hoch-malignem Lymphom war dieses Verfahren erfolgreich, jedoch sind diese Patienten bereits aufgrund ihrer Krankheit und der vorangegangenen Chemotherapie immunsupprimiert. Es ist jedoch davon auszugehen, daß eine derartige Behandlung bei Patienten mit einer schwächeren vorherigen Immunsuppression nicht ausreicht. Nachteil bei diesem Verfahren ist weiterhin, wie bei allen rein unspezifisch immunsuppressiven Agentien, eine erhöhte Infektionsanfälligkeit des behandelten Organismus.

Ein weiteres Verfahren ist die Verwendung von CTLA4, eines Liganden für das kostimulatorische Molekül B7.1. CTLA4 begrenzt u.a. die Aktivierung der T-Zellen auf Antigenstimulation. Die Expression des kostimulatorischen Molekül B7.1 auf antigenpräsentierenden Zellen ist für die Aktivierung von T-Zellen nötig. Bei dem genannten Verfahren wird CTLA4-lgG-Fusionsprotein dem Knochenmark vor der Übertragung zugesetzt, es blockt das kostimulatorische Molekül B7.1 ab, dadurch fehlt den T-Zellen des Empfängers das für die Aktivierung wichtige zweite Signal. Die T-Zellen können nicht reagieren (,Anergie') und ihre Fähigkeit zur akuten Graft-versus-Host Reaktion wird vermindert (9). Dieses Verfahren wurde bei zwölf Patienten erprobt, wovon vier Patienten in einer guten Risikogruppe und acht Patienten in einer Gruppe mit erhöhtem Risiko waren. "Gutes Risiko" bedeutet junge Patienten, wenig Therapieversuche, wenig leukämische Zellen und nicht weit zurückliegender Ausbruch der Krankheit, während diese Verhältnisse bei "erhöhtem Risiko" nicht gegeben sind. Bei dieser Methode müssen jedoch die bestrahlten Blutzellen des Empfängers über eine Inkubationszeit von 36 Stunden hinweg behandelt werden. Neben einer erhöhten Infektionsgefahr ist ein erhöhtes Risiko zur Induktion von Toleranz gegenüber Leukämiezellen des Empfängers zu erwarten.

Aufgabe der vorliegenden Erfindung ist es, eine Stammzelltransplantation auch über HLA-Differenzen hinweg zu ermöglichen, um einerseits auch bei Patienten ohne passenden HLA-identischen Spender eine Stammzelltransplantation zu gewährleisten, was bis jetzt nur mit geringem Erfolg und mit den oben beschriebenen Nachteilen möglich war, und andererseits Toleranz gegenüber transplantierten Organen, die fast immer von HLA-unterschiedlichen Spendern stammen, zu induzieren. Ein weiteres Ziel ist es, ein Präparat hierfür bereitzustellen.

Dies wird durch die Verwendung von Stammzellen und CD6-depletierten Stammzellen zur Herstellung eines pharmazeutischen Zusammensetzung gemäß Anspruch 1 und durch ein Kombinationspräparat gemäß Anspruch 13 erreicht. Bevorzugte Ausführungsformen der Erfindung ergeben sich aus den Unteransprüchen. Menschliche embryonale Stammzellen werden von der Erfindung nicht mit umfaßt.

Figuren:
Fig. 1 zeigt die Suppression zytotoxischer T-Lymphozyten (CTL) durch CD6-negative Stammzellen in Kulturansätzen.
Fig. 2 zeigt ein Modell der Unterdrückung der Alloreaktion durch CD6-negative, CD8-positive Zellen
Fig. 3 ist eine zeitliche Darstellung eines bevorzugten Schemas für die Transplantation gemäß der vorliegenden Erfindung

Tabellen:
Tab. 1 zeigt das Anwachsverhalten von Stammzellen bei HLA-haploidentischer Transplantation und die Graft-versus-Host Krankheit für verschiedene HLA-Differenzen bei erfindungsgemäßen Stammzelltransplantationen und Stammzelltransplantationen nach dem Stand der Technik.
Tab. 2 und 3 zeigen Patientendaten und Behandlungsergebnisse von erfindungsgemäß behandelten Patienten.
Tab. 4 zeigt den Phänotyp bzw. die Zusammensetzung von PBSC nach CD6-Depletion verschiedene Zellpräparate.
Tab. 5 zeigt die Unterdrückung der MLR durch verschiedene Zellpräparate.
Tab. 6 zeigt die Suppressoraktivität von MLR verschiedener Zellpräparate.

### Ausführliche Beschreibung der Erfindung

Gemäß der vorliegenden Erfindung wird eine Zusammensetzung, enthaltend Stammzellen und CD6-depletierte Stammzellen für die zeitlich abgestufte Applikation, zur Toleranzinduktion gegenüber allogenen Transplantaten und/oder zur Behandlung von Leukämie, Blut-, Immun- und/oder Krebserkrankungen verwendet, wobei zunächst die Stammzellen und anschließend die CD6-depletierten Stammzellen appliziert werden. Vorzugsweise werden die CD6-depletierten Zellen vier bis acht Tage, meist sechs Tage, nach der Stammzelltransplantation appliziert. Die Kombination von Stammzellen und CD6-depletierten Stammzellen hat u.a. den Vorteil, daß aufgrund der zweifachen Applikation die Transplantation einer großen Zahl von Stammzellen ermöglicht wird.

Gemäß der vorliegenden Erfindung können die Stammzellen aus dem Knochenmark, aus peripherem Blut oder aus Nabelschnurblut stammen. Diese verschiedenen Stammzellsorten können erfindungsgemäß mit gleichen oder verschiedenen CD6-depletierten Stammzellsorten kombiniert werden. Beispielsweise können zunächst Knochenmarkstammzellen und anschließend CD6-depletierte Knochenmarkstammzellen, CD6-depletierte periphere Blutstammzellen und/oder CD6-depletierte Nabelschnurblutstammzellen appliziert werden. Ebenso sind alle weiteren Kombinationen erfindungsgemäß vorgesehen. Bevorzugt sind die Kombinationen: Knochenmarkstammzellen - CD6-depletierte Knochenmarkstammzellen, Knochenmarkstammzellen - CD6-depletierte periphere Blutstammzellen, periphere Blutstammzellen - CD6-depletierte periphere Blutstammzellen, periphere Blutstammzellen - CD6-depletierte Knochenmarkstammzellen oder Nabelschnurblutstammzellen - CD6-depletierte Nabelschnurblutstammzellen.

Auch können erfindungsgemäß innerhalb eines Transplantats Stammzellen aus verschiedenen Quellen vorliegen, es kann also auch ein Gemisch aus bspw. Knochenmarkstammzellen und peripheren Blutstammzellen und anschließend ein Gemisch aus CD6-depletierten Knochenmarkstammzellen und CD6-depletierten peripheren Blutstammzellen appliziert werden. Auch hier sind erfindungsgemäß sämtliche Kombinationen umfaßt.

Erfindungsgemäß wird der Begriff Stammzellen so verwendet, daß hierunter Stammzellpräparate oder mit Stammzellen angereicherte Präparate oder stammzellreiche Präparate zu verstehen sind. Knochenmark bspw. ist ein "stammzellreiches Präparat". Erfindungsgemäß kann der Begriff Knochenmark synonym zu Knochenmarkstammzellen verwendet werden. Es besteht aber erfindungsgemäß auch die Möglichkeit, daß das Knochenmark vor der Transplantation derart vorbehandelt wird, daß rote Blutkörperchen entfernt werden.

Erfindungsgemäß sind unter peripheren Blutstammzellen solche Stammzellen zu verstehen, die sich in der Peripherie befinden. Durch Verabreichung zumindest eines Zellwachstumsfaktors (z.B. G-CSF, das ist Granulozyten-Colony Stimulating Factor oder GM-CSF) kann erreicht werden, daß Stammzellen aus dem Knochenmark in das Blut mobilisiert werden, ein Verhalten, das sie auch natürlicherweise im Rahmen von schweren Infektionen und Blutverlusten zeigen.

Erfindungsgemäß sind unter Nabelschnurblutstammzellen solche Stammzellen zu verstehen, die nach Abnabelung des neugeborenen Kindes aus der Nabelschnurvene mittels Punktion gewonnen werden.

Es ist bekannt, daß eine größere Zahl von Stammzellen ein besseres Abschneiden der allogenen Transplantation mit sich bringt (10-13). Da bei der vorliegenden Erfindung zweimal eine Applikation von Stammzellen in jeweils der üblichen Menge wie bei sonstigen Stammzelltransplantationen vorgenommen wird, wird die Wirksamkeit des erfinderischen Verfahrens positiv beeinflußt.

Durch die erfindungsgemäße zeitlich versetzte Applikation von Stammzellen und anschließend Stammzellen, die eine Depletion an CD6-positiven T-Zellen aufweisen, wird ermöglicht, daß das Stammzelltransplantat zunächst anwachsen kann. Unter Anwachsen ist zu verstehen, daß die intravenös applizierten Stammzellen mit ihren Homing-Rezeptoren im Knochenmark binden, sich teilen und mit der Produktion von Zellen beginnen.

Üblicherweise erfolgt erst einige Tage nach einer Stammzelltransplantation eine Abstoßung des Transplantates aufgrund einer einsetzenden Immunantwort. Zur Unterdrückung und/oder Verhinderung dieser Immunantwort werden erfindungsgemäß die CD6-depletierten Stammzellen appliziert. Diese weisen eine immunmodulierende Wirkung auf. Während der ersten sechs Tage können bei HLA-Differenz zwischen Spender und Empfänger bereits aktivierte Lymphozyten auftreten, die zur Abstoßung bzw. zur GVH-Reaktion führen können. Erfindungsgemäß können derartige aktivierte Zellen zu dieser Zeit bei Zugabe von CD6-depletierten Stammzellen erkannt und ausgeschaltet werden, ohne daß Stammzellen des Spenders in Mitleidenschaft gezogen werden.

Es wurde im Rahmen dieser Erfindung gefunden, daß Stammzellpräparate mit Depletion von CD6-positiven T-Zellen die Wirkung haben, aktivierte Lymphozyten in ihrer Menge/Proliferation unspezifisch auszuschalten. Über die biologische Bedeutung des CD6-Antigens ist bisher wenig bekannt. Es ist ein Ligand für ALCAM (activated lymphocyte cell adhesion molecule), den Marker aktivierter Lymphozyten. Nach Depletion von CD6-positiven T-Zellen sind fast alle CD4-positiven (z.B. T-Helferzellen) und die meisten CD8-positiven T-Zellen (z.B. zytotoxische T-Zellen) entfernt. Natürliche Killer-Zellen (NK) bleiben übrig.

Die Depletion von CD6-positiven T-Zellen ist seit längerer Zeit für die Prophylaxe von GVH-Krankheit in einzelnen Zentren eingeführt, wobei sowohl bei HLA-identischen (14,15) als auch HLA-differenten (16) Transplantationen GVH-Krankheit in vermindertem Maße auftrat. Bei diesen Verfahren wurden ausschließlich CD6-depletierte Stammzellen transplantiert mit dem Ziel, daß bei CD6-Depletion, wie oben erwähnt, T-Zellen weitgehend aus dem Transplantat entfernt werden. Die Nachteile einer Transplantation ohne T-Zellen wurden eingangs beschrieben. Die vorliegenden Erfindung bezieht sich dagegen auf die supprimierende Aktivität der CD6-depletierten, also der nach CD6-Depletion verbleibenden Zellen.

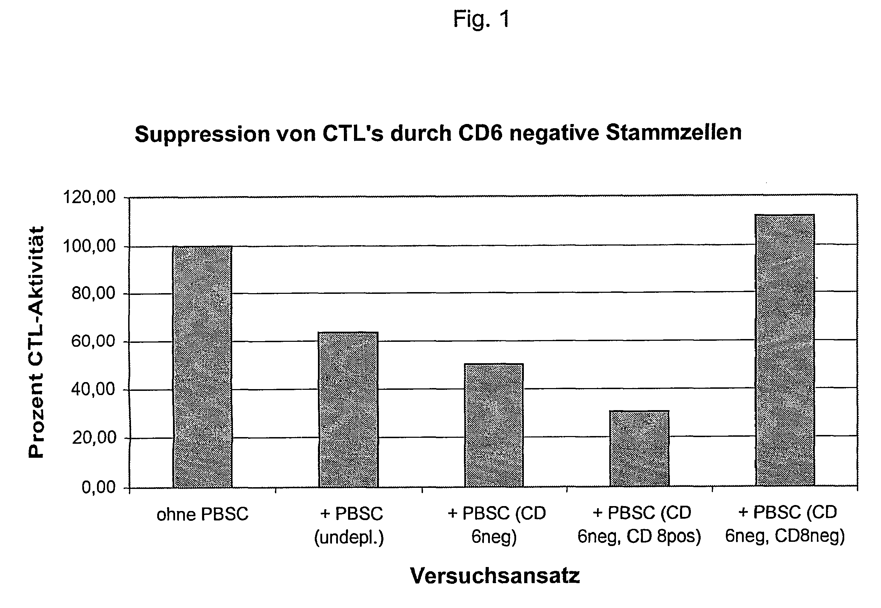

Wie erfindungsgemäß gezeigt werden konnte, unterdrücken CD6-negative Stammzellen in Kultur die Entstehung von zytotoxischen T-Zellen (s. Fig. 1). Fig. 1 zeigt die Suppression zytotoxischer T-Lymphozyten (CTL) durch CD6-negative Stammzellen in Kulturansätzen. Es ist die relative Aktivität zytotoxischer T-Lymphozyten (CTL-Aktivität) für verschiedene Kulturansätze in Abhängigkeit verschiedener peripherer Blutstammzellpräparate (PBSC) dargestellt. In einer gemischten Lymphozytenkultur wurden Lymphozyten eines Spenders (A) bestrahlte Stimulatorzellen eines Spenders (B) zugesetzt (=ohne periphere Blutstammzellen (PBSC)). Dieser Zellsuspension wurden entweder undepletierte PBSC, CD6 depletierte PBSC, die CD8pos. Zellen der nach der CD6 Depletion übrig gebliebenen Zellen oder sowohl CD6 als auch CD8 depletierte PBSC eines Spenders (C) zugegeben. Nach 7 bzw. 14 Tagen wurden die kultivierten Zellen auf ihre CTL-Aktivität hin überprüft. Dazu wurden den CTL's in einem Effektor:Target-Verhältnis von 40:1, 20:1, 10:1, 5:1 und 2:1 mit radioaktivem Chrom markierte Blasten des Stimulators (B) zugegeben. Als Kontrolle dienten Blasten des Effektors (A) und des PBSC-Spenders (C). Gegen (A) und (C) war die CTL-Aktivität jeweils unter 5 Prozent. Die CTL-Aktivität ohne Stammzellbeigabe ist zu 100% angegeben. Bei Zugabe nicht depletierter PBSC sinkt die relative CTL-Aktivität auf ca. 63%. Bei Zugabe CD6-depletierter PBSC sinkt die relative CTL-Aktivität auf ca. 45%. Im Fall der CD6- und CD8-Depletion wird die CTL-Aktivität auf ca. 110% erhöht, während die CD8 positiven Zellen der nach CD6-Depletion übrig gebliebenen Zellen eine weitere Reduktion der relativen CTL-Aktivität auf ca. 30% bewirkt. Die Depletion von NK-Zellen vermindert die suppressive Aktivität nicht. Angereicherte CD34-positive Stammzellen, die sich ebenfalls in der CD6-depletierten Fraktion befinden, haben alleine keine supprimierende Wirkung gezeigt. Erfindungsgemäß konnte gezeigt werden, daß CD6-depletierte Knochenmark- und Blutstammzellpräparate unspezifisch die Immunreaktion gegen allogene Zellen in vitro sowie den Einbau von ³H-Thymidin in der gemischten Lymphozytenkultur und die Generierung zytotoxischer T-Zellen supprimieren. Der supprimierende Effekt wird hauptsächlich durch die nach der CD6-Depletion im Transplantat verbleibenden CD8 positiven Zellen vermittelt.

Neben der supprimierenden Wirkung der CD6-depletierten Stammzell-Präparationen ist der zeitliche Ablauf von Bedeutung. Die CD6-depletierte Fraktion von Stammzellen wird 4 bis 8 Tage, bevorzugt etwa 6 Tage, nach der Stammzelltransplantation verabreicht. Zu diesem Zeitpunkt sind bereits Lymphozyten des Empfängers und des Spenders im Patienten aktiviert, falls HLA-Unterschiede zwischen Spender und Patient bestehen. In der Kultur zeigen die CD6-negativen Stammzellpräparate eine supprimierende Wirkung, die unabhängig von dem HLA-Typ und in gleicher Weise auf Zellen des Spenders, des Patienten und einer dritten Person auftritt. Die unspezifische Wirkung der Stammzellpräparate auf aktivierte T-Zellen spricht gegen individuelle Merkmale wie HLA-Antigene als Zielstrukturen. Vielmehr handelt es sich vermutlich um Strukturen, wie sie allgemein bei der Transformation aktivierter Lymphozyten auftreten.

Die vorliegende Erfindung ermöglicht nun eine Transplantation von Stammzellen und wahlweise zusätzlich von Organen auch über HLA-Differenzen hinweg. Eltern und Kinder des Patienten sind durch Vererbung HLA-haploidentisch, d.h. die Hälfte der HLA-Antigene, die mit einem Chromosomenstück vererbt werden, sind identisch. Identität für Antigene des zweiten HLA-Haplotyps (allgemein werden Antigene für HLA-A, -B und -DR bestimmt; eine erweiterte Bestimmung ist möglich, aber für das Ziel von ungeklärter Relevanz) sind wie bei Unverwandten zufällig. Im statistischen Durchschnitt ist auch die Hälfte der Geschwister HLA-haploidentisch, je ein Viertel ist HLA-identisch bzw. vollständig different mit dem Patienten. Somit bedeutet unsere Erfindung, daß praktisch für jeden Patienten mit Leukämie oder anderen schweren Bluterkrankungen ein Spender in der eigenen Familie gefunden werden kann. Auch bei der Organtransplantation steigt die Wahrscheinlichkeit erheblich, wenn der Spender in 3 HLA-Antigenen mit dem Patienten übereinstimmt. Die Übereinstimmung von mehreren HLA-Antigenen ist vorteilhaft, da wesentliche Körper-eigene Strukturen nur erkannt werden können, wenn sie von eigenen HLA-Antigenen präsentiert werden. Erfindungsgemäß kann die HLA-Differenz auch mehr als 3, theoretisch bis zu 6 von 6 bestimmten Antigenen betragen.

Bei der erfindungsgemäßen Verwendung von Stammzellen und CD6-depletierten Stammzellpräparaten erfahren die Transplantatempfänger eine immunsuppressive Vorbehandlung mit Ganzkörperbestrahlung, Chemotherapie und Antikörpern. Hierdurch wird die Blutbildung und das Immunabwehrsystem des Empfängers fast vollständig zerstört, darüberhinaus kann die immunsuppressive Wirksamkeit von Cyclophosphamid durch Transfusion von Spenderleukozyten vor der Behandlung spezifisch verstärkt werden.

Die Präparation der Knochenmarkstammzellen und die Gewinnung von Blutstammzellen erfolgt erfindungsgemäß nach dem Stand der Technik. Bei Blutgruppenunverträglichkeit werden die roten Blutkörperchen entfernt, um eine Auflösung (Hämolyse) zu vermeiden. Die gewonnenen Stammzellen sollten, wenn möglich direkt vor (bis zu 48 h) der Transplantation gewonnen werden. Sollte dieses nicht möglich sein, so können die Stammzellen auch zu einem früheren Zeitpunkt gewonnen werden und mit einem Kryoprotektanten (z.B. DMSO) versetzt bei bis zu -196°C (in der flüssig- bzw. gasförmigem Stickstoffphase) bis zur Transplantation gelagert werden.

Die Gewinnung von Blutstammzellen erfolgt erfindungsgemäß derart, daß dem Spender zunächst für 4 bis 6 Tage ein Zellwachstumsfaktor (z.B. G-CSF), verabreicht wird, bei einer hohen Zahl von CD34-positiven Stammzellen (etwa > 10/µl Blut) dem Spender mittels einer Zytapherese Blut entnommen wird.

Mit Hilfe eines gegen das CD6-Epitop gerichteten Antikörpers werden die CD6-positiven T-Zellen aus dem Stammzellpräparat entfernt. Nach der Inkubation des gewonnenen Zellapherisats mit einem CD6-Antikörper und dem Auswaschen überschüssiger Antikörper, werden magnetische Partikel (z.B. Eisenpartikel), oder alternativ auch Partikel hoher Dichte (z.B. Nickelpartikel) zugegeben, die sich nur an den CD6-Antikörper anhängen. Die Zell-Antikörper-Suspension wird über einen Magneten geleitet, an dem alle Zellen, die die Antikörper-Partikel-Kombination tragen, gebunden werden. Dichtepartikel-beladene CD6-positive Zellen werden durch Sedimentation abgetrennt. Bei beiden Verfahren beträgt die Wiederfindungsrate der CD6-negativen Zellen bis zu 100%. Die CD6-depletierten Stammzellen sollten, wenn möglich direkt vor (bis zu 48 h) der Transplantation gewonnen werden. Sollte dieses nicht möglich sein, so können die Stammzellen auch zu einen früheren Zeitpunkt gewonnen werden und nach der Separation mit einem Kryoprotektanten (z.B. DMSO) versetzt bei bis zu -196°C (in der flüssig- bzw. gasförmigem Stickstoffphase) bis zur Transplantation gelagert werden.

Die erfindungsgemäß applizierten Stammzellmengen betragen für die nicht CD6-depletierten Stammzellen ungefähr 1 - 4 x 10⁸, bevorzugt 2 - 4 x 10⁸ mononukleäre Zellen/ kg Körpergewicht, angestrebt werden möglichst viele. Im Fall der CD6-depletierten Stammzellen beträgt die Menge ca. 0,4 - 2,0 x 10⁶, bevorzugt 0,8 - 2,0 x 10⁶ CD34-positive Zellen / kg Körpergewicht. Auch hier wird eine möglichst hohe Zellzahl angestrebt.

Sowohl die Knochenmarkstammzellen als auch die Blutstammzellen werden dem Patienten intravenös verabreicht.

Im Fall von Organtransplantationen bieten sich verschiedene Möglichkeiten an, die Erfindung einzusetzen. Bei Lebendspendern (Niere) kann eine immunsuppressive Vorbehandlung ähnlich wie bei Stammzellspendern geplant werden. Nach der Organspende kann der Spender mit Wachstumsfaktor stimuliert und, wie im Falle der Stammzelltransplantation, nach etwa 6 Tagen apherisiert werden. Alternativ kann auch Knochenmark gewonnen werden. Die Präparate werden entweder frisch transfundiert oder nach der Entnahme kryokonserviert. Bei Leichenspendern kann mit der Immunsuppression erst zum Zeitpunkt der Transplantation begonnen werden, die Entnahme von Knochenmark muß gleichzeitig mit der Organentnahme erfolgen. Knochenmark wird nach der CD6-Separation kryokonserviert.

Die Toleranz wird nach Unterdrückung der initialen Abstoßungs- und GVH-Reaktion durch Chimärismus, d.h. Überleben der fremden Blutbildung im Patienten aufrechterhalten. Blut- und Knochenmarkstammzellpräparate können erfindungsgemäß aber auch als Kombinationspräparat vorliegen, da erfindungsgemäß keine HLA-Identität des CD6-depletierten Präparates mit dem Organ- bzw. Stammzelltransplantat notwendig ist. Sofern die HLA-Differenz in dem erfindungsgemäßen Toleranzbereich liegt, kann bei Bedarf auf ein Kombinationspräparat zurückgegriffen werden. Im Falle der Verwendung von Präparaten mehrerer Spender trägt das nicht-depletierte Präparat zum Chimärismus bei.

Erfindungsgemäß ist neben der Tolleranzinduktion gegenüber allogenen Transplantaten die Behandlung von Blut-, Immun- und Krebskrankheiten umfaßt, die auf einer Fehlfunktion der Blutbildung beruhen, beispielsweise nicht maligner Erkrankungen des Bluts (z.B. schwere Aplastische Anämie, Aplastische Anämie, Aplastische Anämie, Sichelzellanämie oder Thalasämie), Immunsystem-Erkrankungen (z.B. Multiple Sklerose, Rheuma (CP), Sklerodermie) und maligner Bluterkrankungen (z.B. akute und chronische Leukämie myeloischen und lymphatischen Ursprungs).

Im folgenden wird die Erfindung anhand von Beispielen erläutert, die jedoch den Schutzbereich der Erfindung nicht einschränken sollen.

### Beispiele

Im Rahmen einer erfindungsgemäßen Studie wurden 21 Patienten mit anderweitig therapierefraktären Leukämien im Rahmen eines Heilversuchs mit einem erfindungsgemäßen Kombinationspräparat aus Knochenmark- bzw. Blutstammzellen und CD6-depletierten Blutstammzellen transplantiert. Es handelte sich um Patienten mit erhöhtem Risiko. Zum Vergleich wurden Patienten in gleichen Leukämie-Stadien herangezogen, bei denen jedoch eine Behandlung gemäß Stand der Technik erfolgte. Es wurden insgesamt 5 Gruppen von Patienten untersucht. Die Ergebnisse sind in der Tabelle I dargestellt.

### 1. Gewinnung der Knochenmarkstammzellen:

Nach einer gründlichen Untersuchung des Spenders auf seine Spendetauglichkeit und der Bestätigung dieser durch den untersuchenden Arzt ca. 3 Wochen vor der geplanten Knochenmarkentnahme wurde den Spendern, in Abhängigkeit der Zellzahl/ml im Knochenmark des Spenders und dem Gewicht des Patienten jeweils ungefähr 1000 - 1500 ml Knochenmark in Vollnarkose aus den beiden Beckenkämmen entnommen und in einem geeigneten Medium (z.B. RPMI 1640) mit Heparin oder Heparin und Zitratlösung (ACD) aufgenommen. Dieses Knochenmark wurde bei Blutgruppenverträglichkeit ohne weitere Behandlung eingesetzt. Falls dies nicht der Fall war, wurde eine Erythrozytendepletion unter Zusatz eines Sedimentationsbeschleunigers mittels Sedimentation oder Zentrifugation durchgeführt.

### 2. Gewinnung der Blutstammzellen:

Nach einer gründlichen Untersuchung des Spenders auf seine Spendetauglichkeit und der Bestätigung dieser durch den untersuchenden Arzt, wird dem Spender ab dem 5. Tag vor der geplanten Stammzellgewinnung der Wachstumsfaktor G-CSF s.c. in einer Dosierung von 5 - 12,5 µg/kg Körpergewicht appliziert. Ab dem 4. Tag wird das Blut des Spenders im Labor auf den Gehalt an CD34 positiven Zellen untersucht. Sobald mehr als 10 CD34 pos. Zellen/µl nachweisbar sind, wird eine Leukozytapherese mittels Zellseparator durchgeführt. Bei der Leukozytapherese wird das Blut außerhalb des Körpers des Spenders mit Hilfe von Heparin und ACD ungerinnbar gemacht. Die stammzellreichen Leukozytenpräparate werden in einem getrennten Blutbeutel mit Gewebekulturmedium (z.B. RPMI 1640) aufgenommen, während das restliche Blut in den Spender zurückfließt. Reichen die gewonnenen Zellzahlen für die Transplantation nicht aus, so wird am nächsten Tag eine weitere Zytapherese durchgeführt, solange es der körperliche Zustand des Spenders zuläßt.

### 3. Gewinnung von Nabelschnurstammzellen:

Nach der Geburt und Abnabelung des neugeborenen Kindes wird durch Punktion der Nabelschnurvene das sich noch in dieser und der Plazenta befindliche Nabelschnurblut, welches sehr reich an Stammzellen ist, gewonnen. Das gewonnene Nabelschnurblut wird durch Sedimentation von einem Großteil der Erythrozyten befreit und anschließend wird durch Zentrifugation ein Teil des Plasmas entfernt. Das so verarbeitete Nabelschnurblut wird auf mindesten zwei Blutbeutel verteilt und nach Zugabe eines Kryoprotektanten bis zum Gebrauch bei -196°C gelagert.

### 4. CD6- Depletion von Stammzellen:

Die gewonnenen Stammzellen werden, nach dem Auswaschen der Thrombozyten durch Zentrifugation, in einer Konzentration von ungefähr 1x10⁸ Zellen/ml mit über dem Sättigunsgbereich liegenden Konzentrationen (∼1 mg/50 x10⁸ Zellen) von anti-CD6-Antikörper (z.B. MT404 von P. Rieber/ G- Riethmüller; andere CD6-Antikörper möglich) für 30 min bei Raumtemperatur inkubiert. Überschüssige, nicht gebundene Antikörper werden durch zweimalige Zugabe von Waschmedium (z.B. RPMI) und Zentrifugieren herausgewaschen. Die Abtrennung der CD6 positiven Zellen erfolgt mit sog. immunomagnetischen Partikeln, das sind Eisenpartikel, die mit einem Antikörper gegen den ersten Antikörper beschichtet sind, z.B einem Schaf-anti-Maus-Antikörper, falls der erste Antikörper von der Maus stammt. Dadurch haften Eisenpartikel an der Oberfläche der CD6-positiven Zelle und erlauben ein Festhalten im Beutel mittels Magnet. Im Falle der Verwendung von magnetischen Partikeln (z.B. Dynabeads®) wird der Blutbeutel nach einer Inkubation von 30 Minuten bei 4°C auf einen Magneten gelegt. Der CD6-negative Inhalt des Beutels wird mittels einer (peristaltischen) Pumpe durch ein Schlauch über einen Sekundärmagneten in einen neuen Blutbeutel überführt. Diese Prozedur wird einmal wiederholt.

Im Falle der Separation mit Mikropartikel hoher Dichte (z.B. Nickelpartikel) werden die CD6-positiven Zellen nicht mit Magnet, sondern mittels Sedimentation entfernt.

Nach der Separation wird die gewonnene Zellsuspension auf Zellzahl und Anteil an CD34-, CD8-, CD4- und CD6-positiven Zellen untersucht.

### 5. Vorbehandlung der Patienten:

Die Vorbehandlung führt insgesamt zu einer schweren Suppression der Blutbildung und Immunabwehrschwäche, wodurch Leukämiezellen möglichst weitgehend eliminiert und die Abstoßungsreaktion gegen das fremde Transplantat möglichst vollständig unterdrückt wird.

Patienten aller Gruppen erhielten zunächst eine Ganzkörperbestrahlung mit je 4 Gy pro Tag an 3 aufeinanderfolgenden Tagen.

Gruppe 1 erhielt zusätzlich lediglich eine Behandlung mit Cyclophosphamid (CY). Dabei wurde den Patienten 0,50 mg CY pro kg Körpergewicht täglich intravenös an vier aufeinanderfolgenden Tagen verabreicht.

Patienten der Gruppe 2 erhielten ebenfalls eine CY-Behandlung wie die der Gruppe 1, jedoch am letzten Tag der Bestrahlung bzw. am Tag vor der ersten CY-Behandlung eine Transfusion von Spenderleukozyten (DBC). Dabei wurde den Patienten jeweils die gesamte Menge Leukozyten verabreicht, die in einer etwa zweistündigen Leukapherese vom Spender gewonnen werden konnte. Diese Leukozyten führen zu einer Stimulierung und Proliferation der Lymphozyten des Patienten, die die Histokompatibilitätsantigene des Spenders erkennen. Die sich teilenden Lymphozyten sind auf die CY-Behandlung, die am nächsten Tag erfolgte besonders empfindlich.

Gruppen 3 erhielt keine Spenderleukozyten, aber 20 mg/kg Kaninchen-anti-Human- Thymozytenglobulin (ATG Fresenius ®) morgens an den Tagen der CY-Behandlung.

Gruppe 4 erhielt die gleiche Vorbehandlung wie die Gruppe 3, allerdings zusätzlich die Spenderleukozytentransfusion am Tage vor der ersten ATG/CY-Behandlung.

Die Gruppe 5 ist die Gruppe, bei der die Behandlung gemäß Patentanmeldung erfolgte. Sie erhielt dieselbe Vorbehandlung wie die Gruppe 4, zusätzlich jedoch 6 Tage nach der Stammzelltransplantation CD6-depletierte Blutstammzellen.

### 6. Stammzelltransplantation:

Am Tage nach Beendigung der jeweiligen Vorbehandlung wurden den Patienten jeweils 1 - 4 x 10⁸ mononukleäre Zellen pro kg Körpergewicht intravenös verabreicht.

### 7. Transplantation CD6-depletierter Stammzellen:

Den Patienten der Gruppe 5 wurden 4 bis 8 Tage nach der Stammzelltransplantation 0,4 - 2 x 10⁶ CD6-depletierte, CD34-positive Stammzellen pro kg Körpergewicht intravenös appliziert.

### 8. GVH-Prophylaxe

Alle Patienten erhielten nach dem Stand der Technik eine prophylaktische immunsuppressive Behandlung mit Cyclosporin A ab Tag -1 und Methotrexat an den Tagen 1, 3 und 6 resp. 7 nach Transplantation intravenös.

Im Rahmen der Versuchsreihe wurden nach der erfindungsgemäßen Methode Patienten behandelt, die mit ihren Spendern in mehreren HLA-Antigenen differierten. Falls der Spender HLA-Antigene besitzt, die dem Patient fremd sind, spricht man von Differenz in Host-versus-Graft Richtung, also in der Richtung der Transplantatabstoßung. Besitzt der Patient HLA-Antigene, die dem Spender fremd sind, spricht man von Differenz in Graft-versus-Host Richtung. Als Differenz wird traditionell der Unterschied in einem serologisch definierten Antigen bewertet. Die neuerdings mit DNA-Testung möglichen Unterschiede auf Gen-Ebene werden nicht gewertet.

### Beispiel: gemeinsamer HLA-Haplotyp kursiv Unterschied in HVG-Richtung: A1 und B5 Unterschied in GVH Richtung: B8 und DR 1

| Spender: | | HLA-Patient: | |
|---|---|---|---|
| A = 2 | A = ***1*** | A = 2 | A = 2 |
| B = 27 | B = ***5*** | B = ***8*** | B = 27 |
| DR = 3 | DR = 3 | DR = ***1*** | DR = 3 |

Die Ergebnisse sind in Tabelle 1 dargestellt. Bei der haploidenten Stammzelltransplantation wurden unterschiedliche Transplantationsmodelle mit unterschiedlicher Konditionierung durchgeführt. Allen gleich ist die Infusion der Stammzellen (Knochenmarkstammzellen oder peripherer Blutstammzellen) am Tage nach Beendigung der Vorbehandlung. In Gruppe II, IV und V erhalten die Patienten nach TBI (Ganzkörperbestrahlung) ein DBC (Spenderleukozytenverabreichung). Patienten der Gruppe V erhalten 4-8 Tage nach Transplantation zusätzlich CD6 depletierte Stammzellen.

Spalte 1 beschreibt die unterschiedlichen Behandlungsgruppen, wobei Gruppe I-IV Vergleichsgruppen zu Gruppe V (erfindungsgemäße Transplantation) sind. Die zweite Spalte gibt die Zahl der in der jeweiligen Gruppe transplantierten Patienten an. Spalte 3 zeigt a) die Anzahl der Mismatche (HLA-Differenzen), b) die Anzahl der Patienten die mit X Mismatchen transplantiert wurden und c) die HLA-Loci, auf denen sich die Mismatche in HVG-Richtung beziehen, d.h. die Merkmale die der Empfänger beim Spender als fremd erkennt. In der Spalte "Anwachsen" wird die Zahl der Patienten angegeben, bei denen ein Anwachsen des Transplantates festgestellt wurde. Die fünfte Spalte entspricht der Spalte 3 nur hier aus der Sicht des Transplantates, d.h. wie viele HLA-Merkmale erkennt das Transplantat beim Empfänger als fremd. In der letzten Spalte steht die Zahl der Patienten, bei denen eine Reaktion des Transplantates gegen den Empfänger festgestellt wurde. Ab Grad II sind diese Reaktionen durch eine zusätzliche Behandlung zu therapieren. Grad IV ist der schwerste GVH-Grad, der meist letal endet.

Trotz größerer HLA-Differenz bei der erfindungsgemäß behandelten Gruppe ist die Quote an Patienten, bei denen der GVH-Grad II oder größer ist, zumindest vergleichbar mit der Quote der nach dem Stand der Technik behandelten Patienten. Es konnte weiterhin festgestellt werden, daß trotz fortgeschrittenerer Leukämie, größerer HLA-Differenz und geringerem Anteil an Kindern die Überlebensrate bei der erfindungsgemäßen Behandlung nicht schlechter war. Weiterhin war es in einer größeren Anzahl von Fällen nicht nötig, nach der Behandlung eine dauerhafte begleitende Immunsuppression vorzunehmen.

In Tab. 2 und 3 sind nähere Informationen zu den in Tab. 1, Gruppe 5, aufgeführten Patienten sowie zu einigen weiteren Patienten angegeben, die zusätzlich behandelt wurden.

Abkürzungen in Tab. 2 und 3:
- AML:: Akute myeloische Leukämie
- NHL:: Non Hodgkin Lymphom
- ALL:: Akute lymphatische Leukämie

- CLL:: Chronisch lymphatische Leukämie
- CML:: Chronisch myeloische Leukämie
- T-ALL:: T-Zell Akute lymphatische Leukämie
- sAML:: sekundäre AML
- OMF:: Osteomyelofibrose
- MDS:: Myelodisplastisches Syndrom
- RA:: refraktäre Anämie
- UPN:: Unbekannte Patientennummer
- Take:: Anwachsen

Ein "-" bei Patient 1138 bedeutet bspw., daß noch kein Anwachsen nach ca. 8 Tagen erfolgt ist, sondern später.

### Weitere Ausführungsbeispiele:

Es geht in der vorliegenden Erfindung um den Einsatz von T-Suppressorzellen oder "Facilitator cells", die nicht nur die Graft-versus-Host Reaktion, sondern auch die Abstoßungsreaktion unterdrücken. Diese Suppressorzellen sind CD6-negativ, d.h. in Anwesenheit von CD6-positiven Zellen sind sie nicht aktiv. Nach CD6-Depletion finden wir eine nahezu vollständige Depletion der CD4-positiven T-Zellen und eine deutliche Depletion der CD8-positiven T-Zellen, von denen aber etwa 4 - 5% übrig bleiben (Tabelle 4). CD34-positive Stammzellen und CD16-positive CD56-positive sog. natürliche Killerzellen bleiben erhalten. Die immunsupprimerende Wirkung dieser CD6-depletierten Zellpräparation wurde in der gemischten Lymphozytenkultur ("mixed leukocyte reaction" - MLR) untersucht, bei der die Lmphozyten von zwei HLA-unterschiedlichen Personen in eine Kulturschale gegeben werden und sich als Folge der Erkennung der unterschiedlichen HLA-D-Determinanten zur Teilung anregen. Um die Richtung der Reaktion zu sehen, werden die Zellen einer Person durch Bestrahlung an der Teilung gehemmt. Gibt man zu der MLR Kochenmark oder sog. Blutstammzellen hinzu, wird die Reaktion geringfügig unterdrückt (Tabelle 5). Setzt man CD6-depletierte Zellpräparationen von Knochenmark oder sog. Blutstammzellen dazu, wird die Reaktion deutlich unterdrückt. Die Unterdrückung der Reaktion ist vermutlich nicht durch CD34-positive Stammzellen verursacht, da positiv selektierte CD34-positive Zellen die Reaktion nur gering unterdrücken (Tabelle 5). Die Unterdrückung der MLR wird weitgehend aufgehoben, wenn zusätzlich zu CD6-positiven Zellen noch die CD8-positiven Zellen entfernt werden. Die Vermutung, daß CD8-positive Zellen, die CD6-negativ sind, für die Suppression verantwortlich sind, wird durch die positive Selektion von CD8-positiven Zellen aus CD6-negativen Präparationen bestätigt (Tabelle 6). Während die CD8-negative Subpopulation von CD6-negativen Zellen kaum unterdrückt, ist die Unterdrückung der CD8-positiven Subpopulation eindrucksvoll. In Tabellen 5 und 6 bedeuten:
cpm - counts per minute (Zähleinheiten pro Minute), gemessen über den Einbau von ³H-Thymidin in Zellen
SI - Stimulationsindex (Faktor)
ARI - Average relative index, mittlerer relativer Index
I, II: verschiedene Versuche

**Tabelle 4**

| **HLA-haploidentische Transplantation Phänotyp der PBSC nach CD 6-Depletion** | | | |
|---|---|---|---|
| | **Zelltyp %** | **Rückgew. (%)** | |
| | Mittel +/- SD | Mittel | Bereich |
| **CD3+/CD4+ (n=10)** | 0.6 +/- 0.4 | 0.5 | 0.2 - 1.4 |
| **CD3+/CD8+ (n=10)** | 4.9 +/- 4.5 | 4.1 | 0.4 - 15.0 |
| **CD3-/CD16 or 56+ (n=10)** | 32.2 +/-13.6 | 26.0 | 12.4 - 54.3 |
| **CD34+ (n=10)** | 69.1 +/-24.6 | 71.2 | 31 - 100 |

**Tabelle 5**

| **HLA-haploidentische Transplantation Phänotyp der PBSC nach CD 6-Depletion** | | | | | | |
|---|---|---|---|---|---|---|
| **Gerrischte Leukocyten Reaktion (MLR)** | | | | | | |
| | **I** | | | **II** | | |
| | cpm | SI | ARI | cpm | SI | ARI |
| **Auto** | 12 504 | 1 | 0% | 28 405 | 1 | 0% |
| **Allo stim** | 44 411 | 3.6 | 100% | 47 397 | 1.7 | 100% |
| **Allo stim. + BM/PBSC**_{**full**} | 19 446 | 1.6 | 21.8% | 33 241 | 1.2 | 25.5% |
| **Allo stim + BM/PBSC**_{**CD6-**} | 12 646 | 1.0 | 0.45% | 25 742 | 0.9 | -14.0% |
| **Allo stim + BM/PBSC**_{**CD34+**} | 34 072 | 2.7 | 67.6% | | n.d. | |
| **Allo stim + BM/PBSC**_{**CD6-/CD8-**} | | **n.d.** | | 37 872 | 1.3 | 50% |

**Tabelle 6**

| **HLA-haploidentische Transplantation** | | | | |
|---|---|---|---|---|
| Suppressoraktivität von MLR ist in der CD6-neg. und CD8-pos. Fraktion | | | | |
| | | cpm | SI | ARI |
| A | | 18.784 | 1 | 0% |
| A* | B | 30.258 | 1.6 | 100% |
| A* | B +PBSC | 20.031 | 1.1 | 11% |
| A* | B +PBSC CD 6- | 11.070 | 0.59 | -67% |
| ***A**** | ***B +PBSC CD 6***^{***-***}*8*^{***+***} | ***8.647*** | ***0.46*** | ***·88%*** |
| A* | B +pBSC CD 6-8- | 17.970 | 0.96 | 0% |
| A | B* +PBSC CD 6- 8- | 29.313 | 1.6 | 44% |
| ***A*** | *B* +PBSC CD 6*^{*-*}8^{*+*} | ***10.519*** | ***0.56*** | ***-35%*** |
| A | B* +PBSC CD 6- | 26.361 | 1.4 | 31.6% |
| A | B* +PBSC | 29.425 | 1.6 | 44% |
| A | B* | 42.698 | 2.3 | 100% |
| B | | 18.821 | 1 | 0% |

Mechanismus, zeitliches Schema und Vorteile der vorliegenden Erfindung:

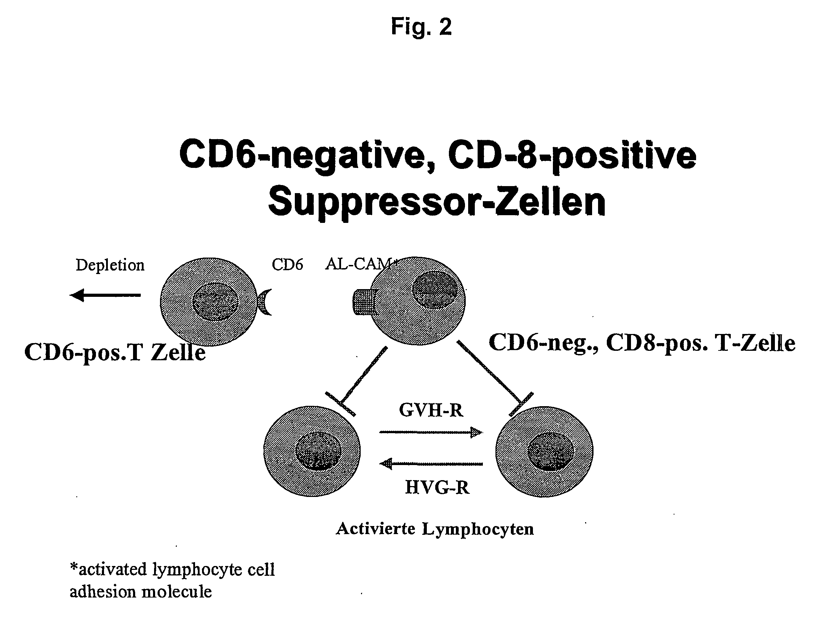

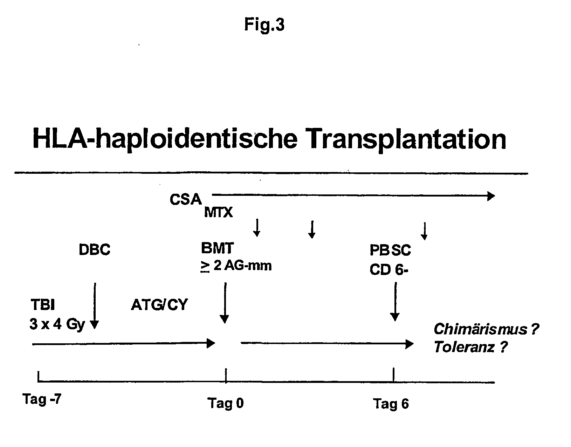

Der Mechanismus der Unterdrückung einer Immunreaktion gegen HLAunterschiedliche Zellen und Organe ist bisher nicht geklärt. Wir wissen lediglich, daß die Unterdrückung nicht spezifisch ist, d.h. CD6-negative, CD8-positive Stammzellpräparate unterdrücken nicht nur die Reaktion fremder Lymphozyten gegen eigene Organe im Sinne einer Graft-versus-Host Reaktion und die Reaktion eigener Lymphozyten gegen fremde Zellen und Organe im Sinne einer Host-versus-Graft- bzw. Abstoßungsreaktion, sondern auch die Reaktion zwischen dritten. Somit kommen nur HLA-unabhängige Reaktionen wie z.B. gegen aktivierte Lymphozyten jeglicher Herkunft in Frage. CD8-positive Suppressorzellen wurden von Reich-Zeliger et al. (17) und Gandy. et al. (18) bei der Maus beschrieben. Beide Forschergruppen haben aber nicht die CD6-Depletion als zielführend beschrieben. Ein Modell, wie CD6-negative, CD8-positive Zellen die Alloreaktion unterdrücken, ist in Figur 2 dargestellt. Der Ligand des CD6-Antigens auf der Zelloberfläche ist ALCAM (activated leukocyte adhesion molecule), CD8-positive Zellen sind zytotoxische Zellen, die in Abwesenheit von CD6-positiven Zellen vermutlich ohne ihren spezifischen T-Zellrezeptor zytotoxisch sein können, wenn die Zielzellen im Rahmen einer Immunreaktion Aktivierungsmarker exprimieren. Die Zytotoxizität solcher Zellen wurde von Reisner et al. nachgewiesen, die als Mediator der Zytotoxizität FAS-L definierten. Da die Aktivierung erst einige Tage nach der Antigenstimulation auftritt, ist die zeitversetzte Gabe (am 6. Tag nach Transplantation) von CD6-depletierten Zellen angezeigt (zeitliches Schema der Transplantation siehe Figur 3).

Abkürzungen in Fig. 3:
TBI - Ganzkörperbestrahlung
DBC - Spenderleukozyten
CSA - Cyclosporin A
MTX - Methotrexat
BMT - Knochenmarktransplantation
ATG - Antithrombocytenglobulin
CY - Cyclophosphamid

Die Transplantationsergebnisse können den Mechanismus nicht beweisen, sondern die Wirkung nur bestätigen. Dabei ist es von wesentlicher Bedeutung, daß unsere Patienten sich mit ihren Spendern in mehr als einem HLA-Antigen unterscheiden. In der Arbeit von Champlin et al. (19) überwiegt der Anteil der Patienten mit unverwandten, HLA-identischen Spendern und verwandten Spendern, die entweder phänotypisch HLA-identisch sind oder sich nur in einem HLA-Antigen unterscheiden. Die Arbeit behandelt im übrigen die Frage der T-Zelldepletion des Transplantates, den Vergleich der Depletion aller T-Zellen mit der Depletion nur eines Teiles der T-Zellen und der Transplantation unbehandelter Präparate. Sie betrifft nicht die zeitversetzte Transplantation Suppressorzell-haltiger Blutstammzellpräparationen. In (20), Craddock et al., Blood 2000; 96: 86-90, ist eine andere Zielstellung betroffen. In dieser Arbeit werden zum Zeitpunkt eines Leukämierückfalls Spenderlymphozyten transfundiert, um die Leukämie zu unterdrücken. Auch die Transplantation CD6+ T-Zell-depletierten Knochenmarks wurde bereits vom Dana Farber Institut berichtet, wobei auch HLA-nicht identische Spender verwendet wurden (16). Allerdings wurde auch in dieser Studie keine zur Knochenmarktransplantation zeitversetzte CD6-depletierte Blutstammzelltransplantation gegeben. Trotz hoher Dosierung der Bestrahlung zur Konditionierung der Patienten (7,5 -10,5 Gy Total Lymphoider Bestrahlung und 14 Gy Ganzkörperbestrahlung) hatten 3 von 27 Patienten kein stabiles Anwachsen. Bei Patienten in einem frühen Krankheitsstadium lag die 2-Jahres-Überlebenswahrscheinlichkeit bei 69%, bei fortgeschritteneren Stadien bei 20%. Im Vergleich hierzu sind unsere im Rahmen von Heilversuchen erzielten Ergebnisse mit weniger Bestrahlung und bei weit fortgeschrittenen Krankheitsstadien bemerkenswert. Bevorzugt erfolgt die Bestrahlung erfindungsgemäß mit etwa 4 Gy, bevorzugt an drei aufeinanderfolgenden Tagen.

Gemäß der vorliegenden Erfindung ist die Verwendung von CD6-depletierten Blutstammzellen sowohl bei der HLA-differenten Transplantation von Knochenmark und Blutstammzellen, als auch bei der Transplantation von Organen wie Niere und Leberlappen von Lebendspendern vorgesehen sowie durchführbar und nachvollziehbar. In beiden Situationen ist der Zeitpunkt der Transplantation auch der Zeitpunkt der ersten Begegnung mit dem unterschiedlichen HLA-Antigen. Damit ist der zeitliche Rahmen für eine zeitversetzte Gabe von CD6-depletierten Blutstammzellen gegeben.

### Literaturliste

1. Prehn RT, Main JM: J. Natl. Cancer Inst. 18: 769-778, 1957
2. Marmont AM, Horowitz MM, Gale RP, et al.: T-cell depletion of HLA-identical transplants in leukemia. Blood; 78: 2120-2130, 1991
3. Martin, PJ, Akatsuka Y, Hahne M et al.: Involvement of donor T-cell effector mechanism in preventing allogeneic marrow graft rejection. Blood, 92, 2177-2181,1998
4. Shapiro R, Starzl TE: Bone marrow augmentation in renal transplant recipients. Transplant Proc 30, 1371-1374, 1998
5. Schumm, M., Günther, W., Kolb, HJ., Rieber, P., Büttner, M., Voss, C., Reitmeier, P., Thierfelder, S., Wilmanns, W. (1994). Prevention of Graft-versus-Host disease in DLA-haplotype mismatched dogs and hemopoietic engraftment of CD6 depleted marrow with and without cG-CSF treatment after transplantation. Tissue antigens, 43: 170-178, 1994
6. Aversa F, Tabilio A, Velardi A, Cunningham 1, Terenzi A, Falzetti F, Ruggeri L, Barbabietola G, Aristei C, Latini P, Reisner Y, Martelli MF: Treatment of highrisk acute leukemia with T-cell-depleted stem cells from related donors with one fully mismatched HLA haplotype. N.Eng.J.Med. 339: 1186-1193, 1998
7. Handgretinger R, Schumm M, Lang P, Greil J, Reiter A, Bader P, Niethammer D, Klingebiel T.:Transplantation of megadoses of purified haploidentical stem cells. Ann N Y Acad Sci. 1999 Apr 30;872:351-61; discussion 361-2.
8. Sykes M, et al.: Mixed lymphohematopoietic chimerism and graft-versuslymphoma effects after non-myeloablative therapy and HLA-mismatched bone marrow transplantation. Lancet 358: 1755-1759, 1999
9. Guinan EC, Boussiotis VA, Neuberg D, Brennan LL, Hirano N, Nadler LM, Gribben JG: Transplantation of anergic histoincompatible bone marrow allografts. N.Eng.J.Med. 340: 1704-1714, 1999
10. Barrett AJ, Ringden O, Zhang MJ, Bashey A, Cahn JY, Cairo MS, Gale RP, Gratwohl A, Locatelli F, Martino R, Schultz KR, Tiberghien P.: Effect of nucleated marrow cell dose on relapse and survival in identical twin bone marrow transplants for leukemia. Blood. 2000 Jun 1;95(11):3323-7.
11. Mavroudis D, Fox M, Read EJ, Carter C, Barrett AJ. Effect of CD34 cell dose on outcome following T-depleted marrow transplantation. Blood. 1996;88:3223
12. Ringdén O, Nilsson B. Death by graft-versus-host disease associated with HLA mismatch, high recipient age, low marrow cell dose and splenectomy. Transplantation. 1985;40:39
13. Paulin T. Importance of bone marrow cell dose in bone marrow transplantation. Clin Transplant. 1992;6:48
14. Soiffer RJ, Ritz J: Selective T cell depletion of donor allogeneic marrow with anti-CD6 monoclonal antibody: rationale and results. Bone Marrow Transplant. 12 Suppl 3: S7-10, 1993
15. Soiffer RJ, Fairclough D, Robertson M, Alyea EP, Anderson K, Freedman AS, Bartlett-Pandite L, Fisher D, Schlossman SF, Stone R, Murray C, Freeman A, Marcus KC, Mauch P, Nadler LM, Ritz J: CD-6 depleted allogeneic bone marrow transplantation for acute leukemia in first complete remission. Blood 89: 3039-3047, 1997
16. Soiffer RJ, Mauch P, Fairclough D, Alyea E, Anderson K, Fisher D, Freedman A, Bartlett-Pandite L, Robertson M, Schlossman R, Gollob J, Marcus K, Murray C, Kuhlman C, Freeman A, Nadler L, Ritz J: CD6+ T cell depleted allogeneic bone marrow transplantation from genotypically HLA nonidentical related donors. Biol.Blood Marrow Transplant. 3: 11-17, 1997
17. Reich_Zeliger S, Zhao Y, Krautghamer R, Bachar-Lustig E, Reisner Y. Anti-Third Party CD8+ CTLs as Potent Veto Cells: Coexpression of CD8 and FasL is a prerequisite.
   Immunity 2000;13: 507-515.
   Gandy KL, Domen J, Aguila H, Weissman IL
   CD8+TCR+ and CD8+TCR- cells on whole bone marrow facilitate engraftment of
   hematopoietic stem cells across allogeneic barriers.. Immunity 1999; 11:579-590.
18. Champlin, R.E. et al, Blood, Vol. 95, No.2 (June), 2000, 3996-4003.
19. Craddock, Ch. et al, Blood, Vol.96, No.1 (July), 2000, 86-90.

**Tab.1: HLA-Inkompatibilität, Anwachsen und Graft-versus-Host Krankheit bei HLA-haploidentischer Transplantation**

| **Behandlungsgruppe** | **Patienten (auswertbar)** | **Unterschiede in HVG-Richtung** | | | **Anwachsen** | **Unterschiede in GVH-Richtung** | | | **GVH ≥ Grad II** |
|---|---|---|---|---|---|---|---|---|---|
| | | **Anzahl Mm** | **Anzahl Pat.** | **Locus Mm** | | **Anzahl Mm** | **Anzahl Pat.** | **Locus Mm** | |
| I. TBI-Cy | | 0 | 1 | | | 0 | 1 | | |
| | 6 | 1 | 5 | 1A; 4DR | 3 | 1 | 5 | 1A; 4DR | 3 |
| | | 2 | 0 | | | 2 | 0 | | |
| | | 3 | 0 | | | 3 | 0 | | |
| II. TBI-DBC-CY | | 0 | 3 | | | 0 | 7 | | |
| | 26 (25) | 1 | 15 | 5A; 2B; 8DR | 24 | 1 | 14 | 6A; 3B; 5DR | 17 |
| | | 2 | 6 | 5A,B; 1A,DR | | 2 | 2 | 1A,B; 1A.DR | |
| | | 3 | 0 | | | 3 | 0 | | |
| III. TBI-ATG-CY | | 0 | 1 | | | 0 | 1 | | |
| | 4 | 1 | 2 | 1B; 1DR | 4 | 1 | 2 | 1B; 1DR | 2 |
| | | 2 | 0 | | | 2 | 0 | | |
| | | 3 | 0 | | | 3 | 0 | | |
| IV. TBI-DBC-ATG-CY | | 0 | 0 | | | 0 | 0 | | |
| | 9 | 1 | 9 | 4A; 2B; 3DR | 9 | 1 | 9 | 4A; 2B; 3DR | 5 |
| | | 2 | 0 | | | 2 | 0 | | |
| | | 3 | 0 | | | 3 | 0 | | |
| V. TBI-DBC-ATG-CY-SC-CD6-neg. | | 0 | 2 | | | 0 | 4 | | |
| | 21 (18) | 1 | 8 | 1A; 4B; 3DR | 18 | 1 | 8 | 2A; 3B, 3DR | 12 |
| | | 2 | 6 | 2A,B; 3A,DR; 1B,DR | | 2 | 5 | 3A,B; 2A,DR | |
| | | 3 | 2 | | | 3 | 1 | | |

| | | | | | | | | | |
|---|---|---|---|---|---|---|---|---|---|
| Abkürzungen: TBI=Ganzkörperbestrahlung; DBC=Spenderleukozyten; CY=Cyclophosfamid; ATG=anti-Thymozyten-Globulin; SC= Stammzellen; CD6-neg.=CD6 depletiert; HVG=Empfänger-gegen-Spender; GVH=Spender-gegen-Empfänger; Mm=mismatch; A,B,DR=HLA-Locus | | | | | | | | | |

**Tab. 2 CD6-depletierte Stammzellen zur Toleranz-Induktion**

| **UPN** | **Initialen** | **Alter Geschlecht** | | **Diagnose** | **Stadium** | **Zytogenetik/Subgruppe** | **Spender** | **Spender Alter Geschlecht** | |
|---|---|---|---|---|---|---|---|---|---|
| 1138 | A.E. | 19,3 | f | AML | Refraktär | Normal | Mutter | 44 | f |
| Zzzz | S.S. | 23,4 | f | NHL | Refraktär | T-NHL CD8 refr. | Schwester | | f |
| 1151 | W.H. | 43,2 | m | ALL | Refraktär | n.a. | Bruder | 54 | m |
| 1863 | E.M. | 51,1 | m | AML | Refraktär | n.a. | Bruder | 53 | m |
| Yyyy | N.V.T. | 39 | m | ALL | refraktär | n.a. | Schwester | 46 | f |
| Xxxx | D.Ü. | 26,3 | f | AML | Refraktär | Trisomie 8 | Schwester | 31 | f |
| 1113 | F.M. | 18,6 | m | AML | Refraktär | Trisomie 8 | Vater | 49 | m |
| 1430 | M.E. | 50,4 | f | CLL | Refraktär | n.a. | Sohn | 31 | m |
| 1409 | B.R. | 40,5 | f | ALL | Refraktär | t (9;22) | Schwester | 37 | f |
| 0867 | S.M. | 53,1 | f | CML | Akzel. Phase | t(9;22) | Neffe | 30 | m |
| 0718 | A.M. | 16,7 | m | T-ALL | Refraktär | Del 9 | Vater | 50 | m |
| 1328 | M.D. | 17,7 | m | AML | Refraktär | 48xy, +8 +21 | Bruder | 35 | m |
| 1480 | F.S.K. | 41,1 | f | sAML | refraktär | Monosomie 7, t(3;3) | Onkel | 65 | m |
| 1813 | S.I. | 35,9 | f | CML | 2.Blastenkrise | 46,XX,t(9;22)(q34;q11)etc. | Vater | 67 | m |
| 1248 | S.H.S. | 38,7 | f | AML | Rezidiv, Chlorome | normal | Bruder | 37 | m |
| 0888 | M.W.F. | 50,4 | m | AML | Refraktär | 46 XY t(10;12) | Schwester | 41 | f |
| 1812 | B.P. | 41,3 | m | AML | Refraktär | Normal | Bruder | 54 | m |
| 0641 | U.V. | 39,1 | m | AML | refraktär | normal | Neffe | 19 | m |
| 0883 | W.A. | 19,2 | m | AML | 1. Rezidiv | t (9;11) | Bruder | 24 | m |
| 1856 | S.F. | 48,6 | m | ALL | Chemorefraktär STI571-Ansprechen | t(9;22), del(7)(p15) del(7)(q11,q2?) | Bruder | 46 | m |
| 1796 | S,O. | 29 | m | AML | Refraktär | n.a. | Mutter | 53 | f |
| 0758 | F.H. | 39 | m | AML | Refraktär | del 7 | Bruder | 33 | m |
| 1692 | S.C. | 31,2 | m | ALL | 1.Remission | t(9;22)(q34;q11), del6(q22); | Mutter | 59 | f |
| 0848 | S.D. | 47,1 | f | CLL | Progredient | n.a. | Tochter | 19 | f |
| 1565 | B.N. | 27,1 | m | AML | Refraktär | Normal | Mutter | 56 | f |
| 1338 | S.W. | 38,7 | m | AML | 1.Remission | t(11;11) | Mutter | 61 | f |
| 1215 | B.B. | 48,4 | f | AML/OMF | Refraktär | n.a. | Kusine | 43 | f |
| 1184 | G.S. | 50,1 | f | MDS | RA | 5q- | Sohn | 15 | m |

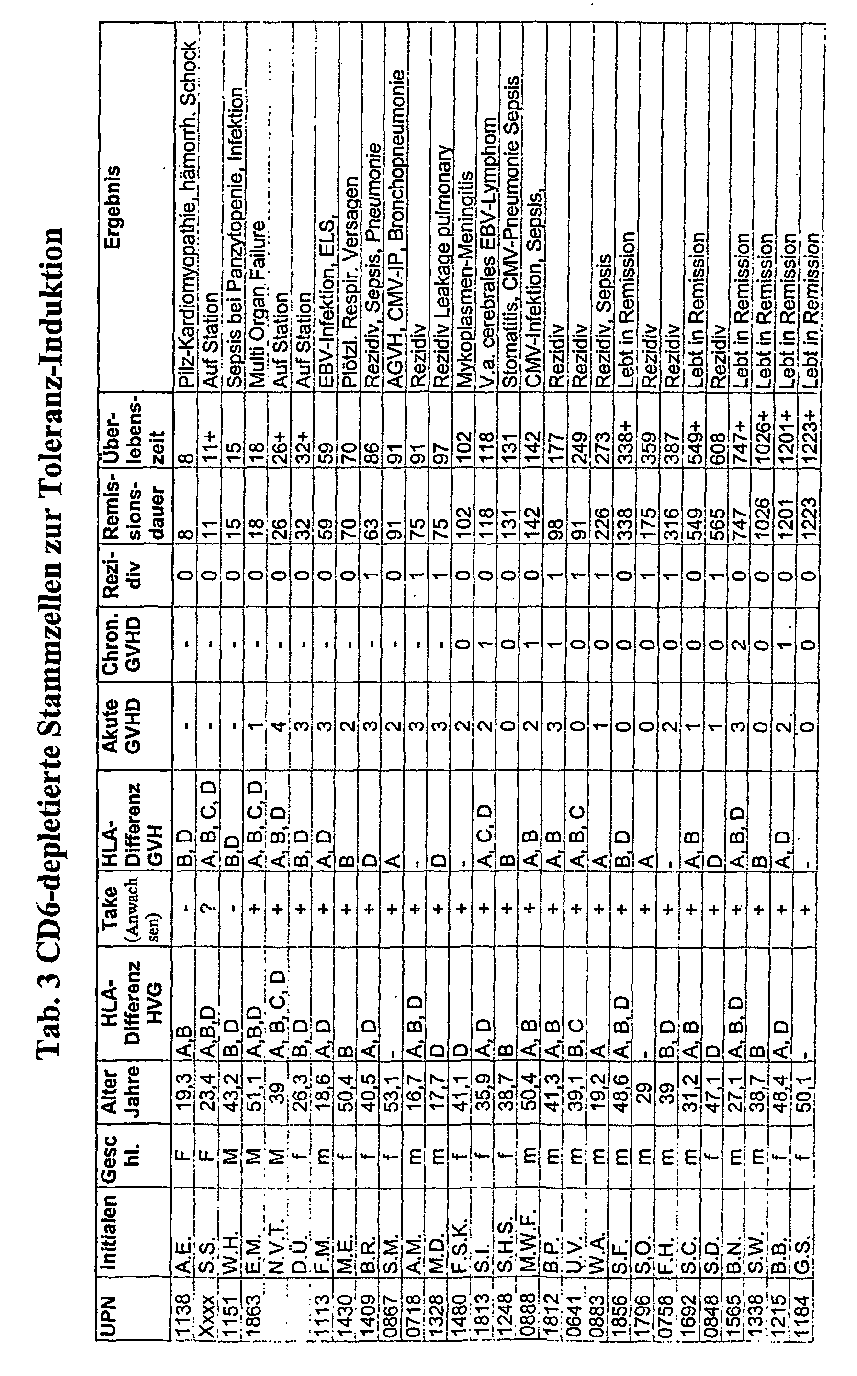

## Patentansprüche

1. Verwendung einer Zusammensetzung, enthaltend Stammzellen und CD6-depletierte Stammzellen zur Herstellung eines pharmazeutischen Zusammensetzung für die zeitversetzte Applikation, zur Toleranzinduktion gegenüber allogenen Transplantaten und/oder zur Behandlung von Blut-, Immun- und/oder Krebserkrankungen,
wobei zunächst die Stammzellen und anschließend die CD6-depletierten Stammzellen appliziert werden, wobei menschliche embryonale Stammzellen ansgenommen sind.

2. Verwendung nach Anspruch 1,
**dadurch gekennzeichnet,**
**daß** die Stammzellen Knochenmarkstammzellen, periphere Blutstammzellen und/oder Nabelschnurblutstammzellen sind, und die CD6-depletierten Stammzellen unabhängig hiervon aus Knochenmarkstammzellen, peripheren Blutstammzellen und/oder Nabelschnurblutstammzellen stammen.

3. Verwendung nach Anspruch 1 oder 2,
**dadurch gekennzeichnet,**
**daß** die CD6-depletierten Stammzellen 4-8 Tage, bevorzugt 6 Tage, nach den Stammzellen appliziert werden.

4. Verwendung nach einem oder mehreren der vorhergehenden Ansprüche,
**dadurch gekennzeichnet,**
**daß** man die CD6-depletierten Stammzellen erhält, indem man die CD6-positiven T-Zellen mit Hilfe eines gegen das CD6-Epitop gerichteten Antikörpers von den Stammzellen oder Stammzell-reichen Präparaten entfernt.

5. Verwendung nach Anspruch 4,
**dadurch gekennzeichnet,**
**daß** an den Antikörper ein magnetisches Partikel gebunden ist, und die CD6-positiven T-Zellen mit Hilfe eines Magneten entfernt werden.

6. Verwendung nach Anspruch 4,
**dadurch gekennzeichnet,**
**daß** an den Antikörper ein Dichtepartikel gebunden ist, und die CD6-positiven T-Zellen sedimentativ entfernt werden.

7. Verwendung nach einem oder mehreren der vorhergehendenAnsprüche,
**dadurch gekennzeichnet,**
**daß** die Stammzellen und die CD6-depletierten Stammzellen intravenös appliziert werden.

8. Verwendung nach einem oder mehreren der vorhergehenden Ansprüche,
**dadurch gekennzeichnet,**
**daß** sich Spender und Empfänger in bis zu 6 HLA-Antigenen, bevorzugt 0 bis 3 HLA-Antigenen unterscheiden.

9. Verwendung nach einem oder mehreren der vorhergehenden Ansprüche,
**dadurch gekennzeichnet,**
**daß** man Stammzellen in einer Menge von 1 -4 x 10⁸, bevorzugt 2-4 x 10⁸ mononukleären Zellen pro kg Körpergewicht,
und CD6-depletierte Stammzellen in einer Menge von 0,4 - 2 x 10⁶, bevorzugt 0,8 - 2 x 10⁶ CD34 positiven Stammzellen pro kg Körpergewicht appliziert.

10. Verwendung nach einem oder mehreren der vorhergehenden Ansprüche,
**dadurch gekennzeichnet,**
**daß** folgende Kombinationen von Stammzellen und CD6-depletierten Stammzellen eingesetzt werden:
Knochenmarkstammzellen - CD6-depletierte Knochenmarkstammzellen,
Knochenmarkstammzellen - CD6-depletierte periphere Blutstammzellen,
periphere Blutstammzellen - CD6-depletierte periphere Blutstammzellen,
periphere Blutstammzellen - CD6-depletierte Knochenmarkstammzellen oder
Nabelschnurblutstammzellen - CD6-depletierte Nabeischnurblutstammzellen.

11. Verwendung nach einem oder mehreren der vorhergehenden Ansprüche,
**dadurch gekennzeichnet,**
**daß** der Stammzellspender und der Spender der CD6-depletierten Stammzellen unterschiedliche Personen sind.

12. Verwendung nach einem oder mehreren der vorhergehenden Ansprüche zur Behandlung von Leukämie.

13. Kombinationspräparat, enthaltend Stammzellen und CD6-depletierte Stammzellen, zur zeitlich abgestuften Anwendung, zur Toleranzinduktion gegenüber Transplantaten und/oder zur Behandlung von Blut-, Immun-, und/oder Krebserkrankungen, wobei menschliche embryonale Stammzellen ansgenommen sind.

14. Kombinationspräparat nach Anspruch 13,
**dadurch gekennzeichnet,**
**daß** die Stammzellen Knochenmarkstammzellen, periphere Blutstammzellen und/oder Nabelschnurblutstammzellen sind, und die CD6-depletierten Stammzellen unabhängig hiervon aus Knochenmarkstammzellen, peripheren Blutstammzellen und/oder Nabelschnurblutstammzellen stammen.

15. Kombinationspräparat nach Anspruch 13 oder 14,
**dadurch gekennzeichnet,**
**daß** die CD6-depletierten Stammzellen 4 - 8 Tage, bevorzugt 6 Tage, nach den Stammzellen appliziert werden.

16. Kombinationspräparat nach einem oder mehreren der Ansprüche 13 bis 15 zur Behandlung von Leukämie.

## Claims

1. The use of a composition containing stem cells as well as CD6 depleted stem cells for the preparation of a pharmaceutical composition for time-staggered application for the induction of tolerance to allogenic transplants and/or for the treatment of blood, immune, and/or cancer diseases wherein first the stem cells are applied followed by application of the CD6 depleted stem cells, wherein human embryonic stem cells are excluded.

2. The use according to claim 1 **characterized in that** said stem cells are bone marrow stem cells, peripheral blood stem cells and/or umbilical cord stem cells, and the CD6 depleted stem cells independently are derived from bone marrow stem cells, peripheral blood stem cells and/or umbilical cord stem cells.

3. The use according to claim 1 or 2 **characterized in that** said CD6 depleted stem cells are applied 4 - 8 days, preferably 6 days after application of the stem cells.

4. The use according to one or more of the preceding claims **characterized in that** said CD6 depleted stem cells are obtained by removing the CD6-positive T cells from the stem cells or stem cell-rich preparations using an antibody directed against the CD6 epitope.

5. The use according to claim 4 **characterized in that** a magnetic particle is bound to the antibody, and the CD6-positive T cells are removed by means of a magnet.

6. The use according to claim 4 **characterized in that** a density particle is bound to the antibody to remove the CD6-positive T cells by sedimentation.

7. The use according to one or more of the preceding claims **characterized in that** said stem cells and CD6 depleted stem cells are applied intravenously.

8. The use according to one or more of the preceding claims **characterized in that** donor and recipient differ from each other in up to 6 HLA antigens, preferably 0 to 3 HLA antigens.

9. The use according to one or more of the preceding claims **characterized in that** stem cells are applied in an amount of 1 - 4 x 10⁸, preferably 2 - 4 x 10⁸ mononuclear cells per kg of body weight, and CD6 depleted stem cells in an amount of 0.4 - 2 x 10⁶, preferably 0.8 - 2 x 10⁶ CD34-positive stem cells per kg of body weight.

10. The use according to one or more of the preceding claims **characterized in that** the following combinations of stem cells and CD6 depleted stem cells are used:
bone marrow stem cells - CD6 depleted bone marrow stem cells, bone marrow stem cells - CD6 depleted peripheral blood stem cells, peripheral blood stem cells - CD6 depleted peripheral blood stem cells, peripheral blood stem cells - CD6 depleted bone marrow stem cells, or umbilical cord blood stem cells - CD6 depleted umbilical cord blood stem cells.

11. The use according to one or more of the preceding claims **characterized in that** the stem cell donor and the donor of the CD6 depleted stem cells are different individuals.

12. The use according to one or more of the preceding claims for the treatment of leucemia.

13. A compound preparation containing stem cells and CD6 depleted stem cells for time staggered application, for the induction of tolerance to transplants and/or for the treatment of blood, immune and/or cancer diseases, wherein human embryonic stem cells are excluded.

14. The compound preparation according to claim 13 **characterized in that** said stem cells are bone marrow stem cells, peripheral blood stem cells and/or umbilical cord stem cells, and said CD6 depleted stem cells independently are derived from bone marrow stem cells, peripheral blood stem cells and/or umbilical cord stem cells.

15. The compound preparation according to claim 13 or 14 **characterized in that** said CD6 depleted stem cells are applied 4 - 8 days, preferably 6 days after application of the stem cells.

16. The compound preparation according to one or more of claims 13 to 15 for the treatment of leucemia.

## Revendications

1. Utilisation d'une composition contenant des cellules souches et des cellules souches dépourvues de CD6, pour produire une composition pharmaceutique pour l'application retardée, afin d'induire une tolérance à l'encontre de greffons allogènes et/ou pour le traitement d'affections hématologiques, immunologiques et/ou de cancers, dans laquelle on applique tout d'abord les cellules souches et ensuite les cellules souches dépourvues de CD6, les cellules souches embryonnaires humaines étant exclues.

2. Utilisation selon la revendication 1, **caractérisée en ce que** les cellules souches proviennent de cellules souches de moelle osseuse, de cellules souches de sang périphérique et/ou de cellules souches de sang de cordon ombilical, et les cellules souches dépourvues de CD6 proviennent indépendamment de celles-ci de cellules souches de moelle osseuse, de cellules souches de sang périphérique et/ou de cellules souches de sang de cordon ombilical.

3. Utilisation selon la revendication 1 ou 2, **caractérisée en ce que** les cellules souches dépourvues de CD6 sont appliquées 4 à 8 jours, de préférence 6 jours, après les cellules souches.

4. Utilisation selon une ou plusieurs des revendications précédentes, **caractérisée en ce que** l'on obtient les cellules souches dépourvues de CD6, en éliminant les lymphocytes T positifs à CD6 à l'aide d'un anticorps dirigé contre l'épitope de CD6 des cellules souches ou de préparations riches en cellules souches.

5. Utilisation selon la revendication 4, **caractérisée en ce qu'**une particule magnétique est liée à l'anticorps et les lymphocytes T positifs à CD6 sont éliminés à l'aide d'un aimant.

6. Utilisation selon la revendication 4, **caractérisée en ce qu'**une particule de haute densité est liée à l'anticorps, et les lymphocytes T positifs à CD6 sont éliminés par sédimentation.

7. Utilisation selon l'une quelconque des revendications précédentes, **caractérisée en ce que** les cellules souches et les cellules souches dépourvues de CD6 sont appliquées par voie intraveineuse.

8. Utilisation selon l'une quelconque des revendications précédentes, **caractérisée en ce que** donneur et receveur se distinguent par jusqu'à 6 antigènes de HLA, de préférence de 0 à 3 antigènes de HLA.

9. Utilisation selon l'une ou plusieurs des revendications précédentes, **caractérisée en ce** l'on applique les cellules souches en une quantité de 1 à 4 x 10⁸, de préférence 2 à 4 x 10⁸ cellules mononucléaires par kg de poids corporel et les cellules souches dépourvues de CD6 en une quantité de 0,4 à 2 x 10⁶, de préférence de 0,8 à 2 x 10⁶ cellules souches positives à CD34 par kg de poids corporel.

10. Utilisation selon l'une ou plusieurs des revendications précédentes, **caractérisée en ce que** les combinaisons suivantes de cellules souches et de cellules souches dépourvues de CD6 sont utilisées :
cellules souches de moelle osseuse - cellules souches de moelle osseuse dépourvues de CD6,
cellules souches de moelle osseuse - cellules souches de sang périphérique dépourvues de CD6,
cellules souches de sang périphérique - cellules souches de sang périphérique dépourvues de CD6,
cellules souches de sang périphérique - cellules souches de moelle osseuse dépourvues de CD6, ou
cellules souches de sang de cordon ombilical - cellules souches de sang de cordon ombilical dépourvues de CD6.

11. Utilisation selon l'une ou plusieurs des revendications précédentes, **caractérisée en ce que** le donneur de cellules souches et le donneur de cellules souches dépourvues de CD6 sont des personnes différentes.

12. Utilisation selon l'une ou plusieurs des revendications précédentes, pour le traitement de la leucémie.

13. Préparation combinée, contenant des cellules souches et des cellules souches dépourvues de CD6, pour utilisation échelonnée dans le temps, pour induire une tolérance vis-à-vis de greffons et/ou pour le traitement d'affections hématologiques, immunologiques et/ou de cancers, les cellules souches embryonnaires humaines étant exclues.

14. Préparation combinée selon la revendication 13, **caractérisée en ce que** les cellules souches sont des cellules souches de moelle osseuse, des cellules souches de sang périphérique et/ou des cellules souches de sang de cordon ombilical, et les cellules souches dépourvues de CD6 indépendamment de celles-ci proviennent de cellules souches de moelle osseuse, de cellules souches de sang périphérique et/ou de cellules souches de sang de cordon ombilical.

15. Préparation combinée selon la revendication 13 ou 14, **caractérisée en ce que** les cellules souches dépourvues de CD6 sont appliquées 4 à 8 jours, de préférence 6 jours après les cellules souches.

16. Préparation combinée selon l'une ou plusieurs des revendications 13 à 15, pour le traitement de la leucémie.
